# EUROPEAN PATENT APPLICATION

(11) **EP 4 533 998 A1**
(43) Date of publication of application: **09.04.2025**
(21) Application number: 23815830.7
(22) Date of filing: 19.05.2023
(51) Int. Cl.: A61B 1/045

(54) **IMAGE PROCESSING DEVICE, IMAGE PROCESSING METHOD, AND STORAGE MEDIUM**

(30) Priority: 30.05.2022 WO PCT/JP2022/021896
(71) Applicant: NEC Corporation, 108-8001 Tokyo (JP)
(72) Inventor: IWADATE, Yuji, Tokyo 108-8001 (JP)
(74) Representative: Betten & Resch
(86) International application number: PCT/JP2023/018737
(87) International publication number: WO 2023/234071

(57) **Abstract**

The image processing device 1X includes an acquisition means 31X, a selection means 32X, and a determination means 33X. The acquisition means 31X is configured to acquire data obtained by applying Fourier transform to an endoscopic image of an examination target photographed by a photographing unit provided in an endoscope. The selection means 32X is configured to select partial data that is a part of the data. The determination means 33X is configured to make a determination regarding an attention point to be noticed in the examination target based on the partial data. It can be used for assisting user's decision making,

## Description

### TECHNICAL FIELD

The present disclosure relates to a technical field of an image processing device, an image processing method, and a storage medium for processing an image to be acquired in endoscopic examination.

### BACKGROUND

An endoscopic examination system for displaying images taken in the lumen of an organ is known. For example, Patent Literature 1 discloses an endoscopic examination system that detects a target region based on an endoscopic image and a target region detection threshold value and that determines whether the target region is either a flat lesion or a raised lesion. Further, Patent Literature 2 discloses an image processing device which generates a tomographic image by applying the inverse Fourier transform to k-space data obtained by an MRI device.

### CITATION LIST

### PATENT LITERATURE

Patent Literature 1: WO2019/146077
Patent Literature 2: JP2021-041065A

### SUMMARY

### PROBLEM TO BE SOLVED

The determination process of an attention point such as a lesion part from an image captured in endoscopic examination should be done on a real-time basis. Considering the possibility of performing other complicated processes such as a further process using the determination result, it is desirable to reduce the amount of calculation required for the above-described determination process.

In view of the above-described issue, it is therefore an example object of the present disclosure to provide an image processing device, an image processing method, and a storage medium capable of making a determination regarding an attention point while suppressing an increase in the amount of calculation in endoscopic examination.

### MEANS FOR SOLVING THE PROBLEM

One mode of the image processing device is an image processing device including:
an acquisition means configured to acquire data obtained by applying Fourier transform to an endoscopic image of an examination target photographed by a photographing unit provided in an endoscope;
a selection means configured to select partial data that is a part of the data; and
a determination means configured to make a determination regarding an attention point to be noticed in the examination target based on the partial data.

One mode of the image processing method is an image processing method executed by a computer, the image processing method including:
acquiring data obtained by applying Fourier transform to an endoscopic image of an examination target photographed by a photographing unit provided in an endoscope;
selecting partial data that is a part of the data; and
making a determination regarding an attention point to be noticed in the examination target based on the partial data.

One mode of the storage medium is a storage medium storing a program executed by a computer, the program causing the computer to:
acquire data obtained by applying Fourier transform to an endoscopic image of an examination target photographed by a photographing unit provided in an endoscope;
select partial data that is a part of the data; and
make a determination regarding an attention point to be noticed in the examination target based on the partial data.

### EFFECT

An example advantage according to the present invention is to suitably make a determination regarding an attention point while suppressing an increase in the amount of calculation in endoscopic examination.

### BRIEF DESCRIPTION OF THE DRAWINGS

[FIG. 1] It illustrates a schematic configuration of an endoscopic examination system.
[FIG. 2] It illustrates a hardware configuration of an image processing device.
[FIG. 3] It is a diagram showing an outline of a lesion determination process.
[FIG. 4] It is a functional block diagram of the image processing device relating to the lesion determination process.
[FIG. 5] FIGS. 5A to 5E each indicates a specific example with clear indication of a selected area to be selected as partial data in k-space data and a non-selected area.
[FIG. 6] It illustrates a graph of the accuracy rate in the experiment using the partial data and the k-space data shown in FIG. 5A to FIG. 5E in endoscopic examination.
[FIG. 7] FIGS. 7A to 7C each illustrates an example of the k-space data with clear indication of the selected area and the non-selected area in a case where the non-selected areas are provided for both of the range of the k-x axis and the range of the part of k-y axis.
[FIG. 8] It illustrates an example of the display screen image displayed by a display device in endoscopic examination.
[FIG. 9] It illustrates an example of a flowchart showing an outline of a process performed by the image processing device in endoscopic examination in the first example embodiment.
[FIG. 10] It is a schematic configuration diagram of an endoscopic examination system according to a modification.
[FIG. 11] It is a block diagram of an image processing device according to a second example embodiment.
[FIG. 12] It illustrates an example of a flowchart executed by the image processing device in the second example embodiment.

### EXAMPLE EMBODIMENTS

Hereinafter, example embodiments of an image processing device, an image processing method, and a storage medium will be described with reference to the drawings.

### <First Example Embodiment>

### (1) System Configuration

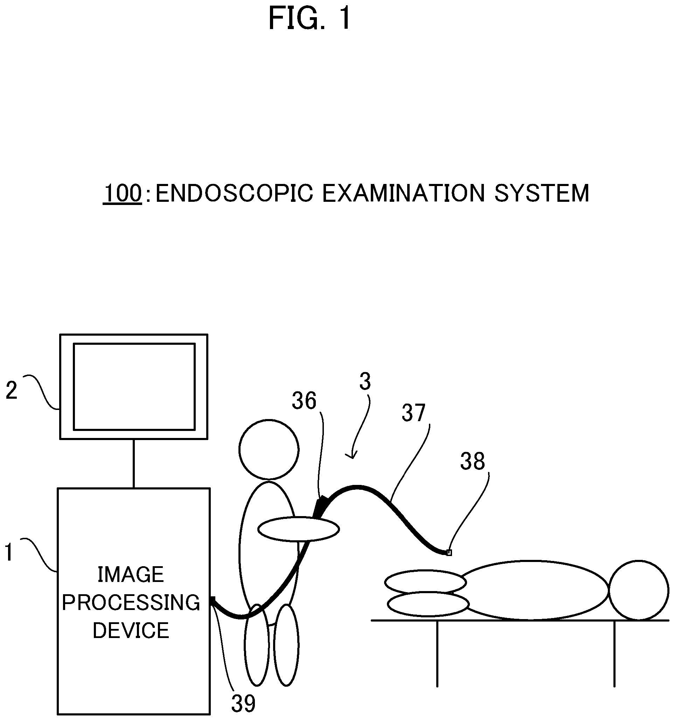

FIG. 1 shows a schematic configuration of an endoscopic examination system 100. As shown in FIG. 1, an endoscopic examination system 100 is a system for presenting information relating to a lesion part (also referred to as "lesion part") of examination target which is suspected of lesion to an examiner such as a doctor who performs examination or treatment using an endoscope, and mainly includes an image processing device 1, a display device 2, and an endoscope 3 connected to the image processing device 1. The lesion part is an example of the "attention point".

The image processing device 1 acquires an image (also referred to as "endoscopic image Ia") captured by the endoscope 3 in time series from the endoscope 3 and displays a screen image based on the endoscopic image Ia on the display device 2. The endoscopic image Ia is an image captured at predetermined time intervals in at least one of the insertion process of the endoscope 3 to the subject or the ejection process of the endoscope 3 from the subject. In the present example embodiment, the image processing device 1 analyzes the endoscopic image Ia to determine the presence or absence of the lesion part in the endoscopic image Ia, and displays the information on the determination result on the display device 2.

The display device 2 is a display or the like for display information based on the display signal supplied from the image processing device 1.

The endoscope 3 mainly includes an operation unit 36 for examiner to perform a predetermined input, a shaft 37 which has flexibility and which is inserted into the organ to be photographed of the subject, a pointed end unit 38 having a built-in photographing unit such as an ultra-small image pickup device, and a connecting unit 39 for connecting with the image processing device 1.

The configuration of the endoscopic examination system 100 shown in FIG.1 is an example, and various change may be applied thereto. For example, the image processing device 1 may be configured integrally with the display device 2. In another example, the image processing device 1 may be configured by a plurality of devices.

It is noted that the target of the endoscopic examination in the present disclosure is not limited to a large bowel, it may be any organ subject to endoscopic examination such as esophagus, stomach, pancreas. Examples of the target of the endoscopic examination in the present disclosure include a laryngendoscope, a bronchoscope, an upper digestive tube endoscope, a duodenum endoscope, a small bowel endoscope, a large bowel endoscope, a capsule endoscope, a thoracoscope, a laparoscope, a cystoscope, a cholangioscope, an arthroscope, a spinal endoscope, a blood vessel endoscope, and an epidural endoscope. In addition, the conditions of the lesion part to be detected in endoscopic examination are exemplified as (a) to (f) below.
(a) Head and neck: pharyngeal cancer, malignant lymphoma, papilloma
(b) Esophagus: esophageal cancer, esophagitis, esophageal hiatal hernia, Barrett's esophagus, esophageal varices, esophageal achalasia, esophageal submucosal tumor, esophageal benign tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small bowel: small bowel cancer, small bowel neoplastic disease, small bowel inflammatory disease, small bowel vascular disease
(f) Large bowel: colorectal cancer, colorectal neoplastic disease, colorectal inflammatory disease; colorectal polyps, colorectal polyposis, Crohn's disease, colitis, intestinal tuberculosis, hemorrhoids.

### (2) Hardware Configuration

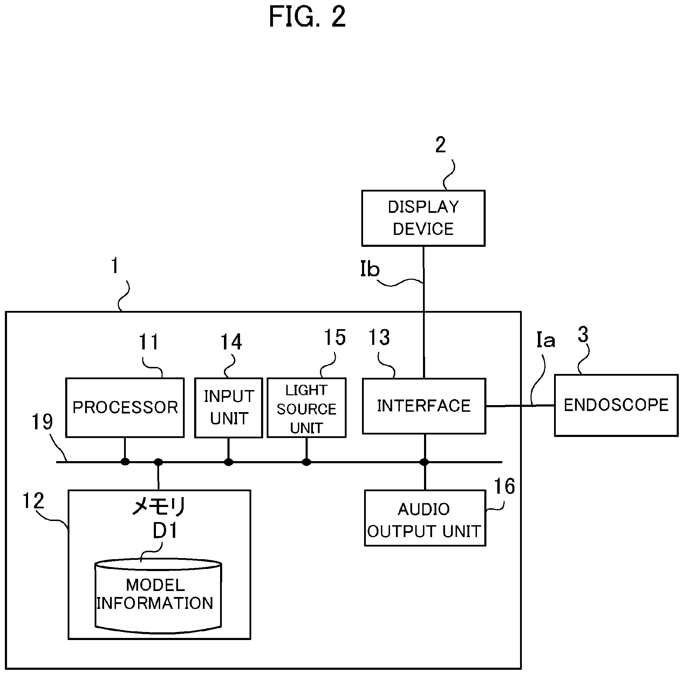

FIG. 2 shows the hardware configuration of the image processing device 1. The image processing device 1 mainly includes a processor 11, a memory 12, an interface 13, an input unit 14, a light source unit 15, and an audio output unit 16. Each of these elements is connected via a data bus 19.

The processor 11 executes a predetermined process by executing a program or the like stored in the memory 12. The processor 11 is one or more processors such as a CPU (Central Processing Unit), a GPU (Graphics Processing Unit), and a TPU (Tensor Processing Unit). The processor 11 is an example of a computer.

The memory 12 is configured by a variety of volatile memories which is used as working memories, and nonvolatile memories which stores information necessary for the process to be executed by the image processing device 1, such as a RAM (Random Access Memory) and a ROM (Read Only Memory). The memory 12 may include an external storage device such as a hard disk connected to or built in to the image processing device 1, or may include a storage medium such as a removable flash memory. The memory 12 stores a program for the image processing device 1 to execute each process in the present example embodiment.

The memory 12 also stores model information D1. The model information D1 is the information regarding a lesion determination model configured to output a determination result regarding a lesion part in the endoscopic image. The model informational D1 contains the parameters required to configure a lesion determination model.

The lesion determination model is, for example, a model trained to output a determination result regarding the lesion part in the endoscopic image in response to input, to the model, of input data based on the endoscopic image. In other words, the lesion determination model is a model which learned a relation between input data inputted to the lesion determination model and the determination result regarding the lesion part in the endoscopic image used for the generation of the input data. The lesion determination model may be a model configured to determine at least one of the presence or absence of a particular type of disease and/or the degree of the disease, or may be a model configured to determine at least the type of the detected disease. For example, the lesion determination model may be configured to determine the degree of inflammation or the amount of bleeding of the photographed part in the endoscopic image. Instead of or in addition to the above-described determination result, the lesion determination model may be configured to output information indicating the position or region (area) of the lesion part in the inputted endoscopic image.

A supplementary description will be herein given of the learning of the lesion determination model. The lesion determination model is trained in advance on the basis of a set of input data which conforms to the input format of the lesion determination model and corresponding correct answer data indicating the determination result of the correct answer regarding the lesion part in the endoscopic image used for generating the input data. As will be described later, the data to be inputted to the lesion determination model is data corresponding to a frequency domain selected from the k-space data obtained by applying the Fourier transformation to the endoscopic image. Here, the lesion determination model may be, for example, any machine learning model (including a statistical model, hereinafter the same) such as a neural network and a support vector machine. Examples of typical models of such neural networks include Fully Convolutional Network, SegNet, U-Net, V-Net, Feature Pyramid Network, Mask R-CNN, and DeepLab. When the lesion determination model includes a neural network, the model information D1 includes various parameters such as, for example, a layer structure, a neuron structure of each layer, the number of filters and the size of filters in each layer, and the weight for each element of each filter.

It is noted that the lesion determination model is not limited to being a machine-learning model, and may be a model for determining the presence or absence of a lesion part caused by a particular disease based on the proportions of red, green, and blue (RGB) in the inputted endoscopic image or the like. For example, the lesion determination model may be a model that determines that there is a lesion part based on a particular disease (e.g., inflammation) if the proportion (e.g., the averaged proportion in all pixels) of red in RGB in the inputted endoscopic image is equal to or greater than a predetermined threshold value. In this instance, the above-described calculation formula and threshold value for calculating the proportion of red are stored in advance in the memory 12 as the model information D1. In addition, when there are a plurality of target diseases, the lesion determination model may be provided for each target disease. In this situation, the parameters for configuring the lesion determination model for each target disease are stored in the model information D1.

The interface 13 performs an interface operation between the image processing device 1 and an external device. For example, the interface 13 supplies the display information "Ib" generated by the processor 11 to the display device 2. Further, the interface 13 supplies the light generated by the light source unit 15 to the endoscope 3. The interface 13 also provides an electrical signal to the processor 11 indicative of the endoscopic image Ia supplied from the endoscope 3. The interface 13 may be a communication interface, such as a network adapter, for wired or wireless communication with the external device, or a hardware interface compliant with a USB (Universal Serial Bus), a SATA (Serial AT Attachment), or the like.

The input unit 14 generates an input signal based on the operation by the examiner. Examples of the input unit 14 include a button, a touch panel, a remote controller, and a voice input device. The light source unit 15 generates light for supplying to the pointed end unit 38 of the endoscope 3. The light source unit 15 may also incorporate a pump or the like for delivering water and air to be supplied to the endoscope 3. The audio output unit 16 outputs a sound under the control of the processor 11.

### (4) Lesion Determination Process

A description will be given of a lesion determination process that is a process relating to determination of the lesion part.

### (4-1) Outline

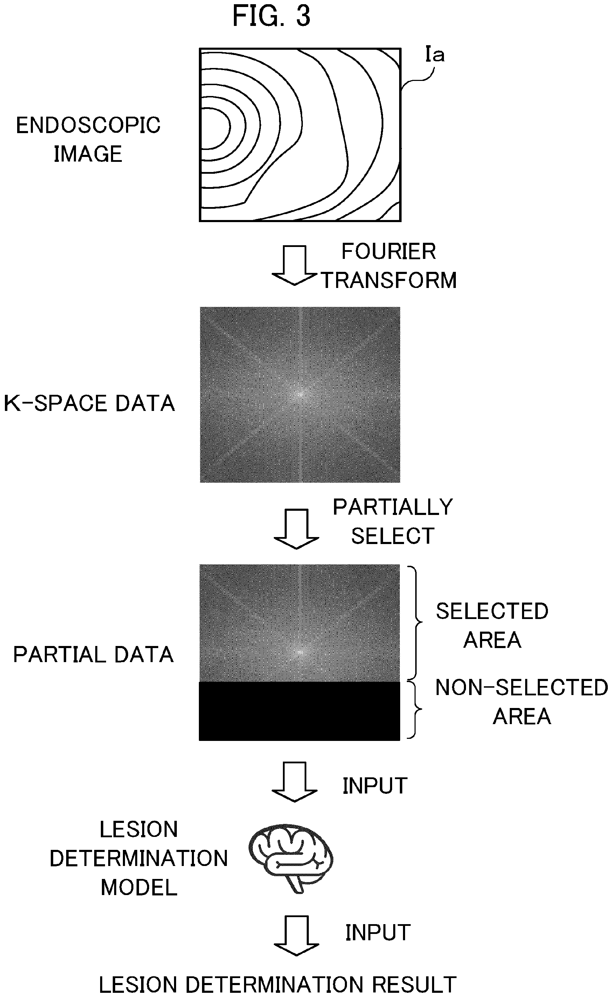

FIG. 3 is a diagram illustrating an outline of a lesion determination process that is executed by the image processing device 1.

The image processing device 1 firstly generates data (also referred to as "k-space data") in k-space by applying the Fourier transform (specifically, the two-dimensional Fourier transform in the vertical direction and the horizontal direction of the image) to an endoscopic image Ia acquired during endoscopic examination. Hereafter, the position coordinates in the real space corresponding to the horizontal and vertical axes of the endoscopic image Ia will be denoted as "(x, y)", and the coordinates of the spatial frequency corresponding to the horizontal and vertical axes of the k-space data will be denoted as "(kx, ky)".

For example, the k-space data confirms to the image format (third-order tensor) and is the data obtained by converting data representing a complex number for each spatial frequency into the absolute value for each spatial frequency, wherein the data representing the complex number for each spatial frequency is obtained by applying the Fourier transform to the endoscopic image Ia. The k-space data may be data obtained by applying the Fourier transform to the endoscopic image Ia itself (i.e., data representing the complex number for each spatial frequency) or may be data representing the argument (i.e., phase) for each spatial frequency into which the data obtained by applying the Fourier transform to the endoscopic image Ia is converted. The k-space data may be data obtained by applying the logarithmic conversion to the value (complex number, absolute value, or phase) for each spatial frequency. In the k-space data shown in FIG. 3, the higher the value for each spatial frequency is, the higher the brightness at the each spatial frequency becomes.

Next, the image processing device 1 selects, from the k-space data, data (also referred to as "partial data") in a part of the frequency domain on the k-space where the k-space data is expressed. The partial data is the data in the image format (third order tensor), and it becomes the data which matches the input format of the lesion determination model. In the example of FIG. 3, as an example, the image processing device 1 generates partial data in which the upper 3/4 range (value range) along the k-y axis where the k-space data exists is selected. In FIG. 3, the "selected area" denotes the frequency domain in the k-space selected from the k-space data as the partial data, and the "non-selected area" denotes the frequency domain in the k-space not selected from the k-space data as the partial data. Thus, the image processing device 1 suitably reduces the amount of data to be used for determining the lesion part.

Then, the image processing device 1 inputs the partial data to the lesion determination model to which the learned parameters stored in the model information D1 is applied, and acquires a determination result (lesion determination result) relating to the lesion part which is outputted by the lesion determination model in response to the input. Then, the image processing device 1 performs the process for displaying the information based on the lesion determination result outputted by the lesion determination model on the display device 2, the process for making a further determination (including automatic diagnosis) regarding the lesion part based on the lesion determination result, and the like.

By performing such a process, the image processing device 1 can reduce the amount of calculation required to make a determination regarding the lesion part while maintaining the determination accuracy regarding the lesion part. Thus, even in the case of performing additional processing based on the lesion determination result, the image processing device 1 can reduce the processing amount required for generating the lesion determination result thereby to ensure the real-time processing.

### (4-2) Functional Blocks

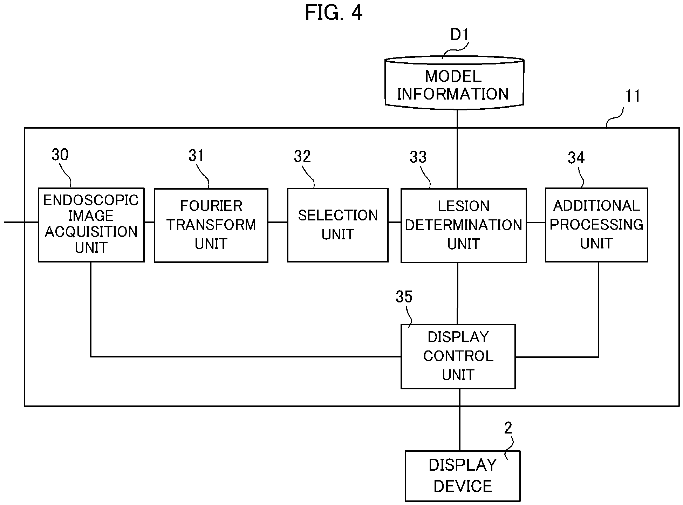

FIG. 4 is a functional block diagram of the image processing device 1 related to the lesion detection process. The processor 11 of the image processing device 1 functionally includes an endoscopic image acquisition unit 30, a Fourier transform unit 31, a selection unit 32, a lesion determination unit 33, an additional processing unit 34, and a display control unit 35. In FIG. 4, blocks to exchange data with each other are connected to each other by a solid line, but the combination of blocks to exchange data with each other is not limited thereto. The same applies to other functional block diagrams described below.

The endoscopic image acquisition unit 30 acquires an endoscopic image Ia taken by the endoscope 3 through the interface 13 at predetermined intervals. The endoscopic image acquisition unit 30 supplies the acquired endoscopic image Ia to the Fourier transform unit 31 and the display control unit 35, respectively.

The Fourier transform unit 31 generates k-space data obtained by applying the Fourier transform to the endoscopic image Ia supplied from the endoscopic image acquisition unit 30. It is noted that the Fourier transform unit 31 may generate, as the k-space data, at least one of: data representing an absolute value or phase for each spatial frequency into which data representing the complex number for each spatial frequency is converted; and/or data obtained by applying logarithmic conversion to the value for each spatial frequency, after applying the Fourier transform to the endoscopic image Ia.

The selection unit 32 selects, from the k-space data, the partial data that is data in a part of the frequency domain in the k-space where the k-space data generated by the Fourier transform unit 31 is present, as partial data. The selection approach by the selection unit 32 will be described later.

The lesion determination unit 33 makes a determination regarding the lesion part in the endoscopic image Ia that is the source of the partial data, based on the partial data generated by the selection unit 32, and then supplies information (also referred to as "lesion determination information") indicating the lesion determination result to the additional processing unit 34 and the display control unit 35. In this case, the lesion determination unit 33 inputs the partial data supplied from the selection unit 32 to the lesion determination model configured by referring to the model information D1, and generates the lesion determination information based on the lesion determination result outputted by the lesion determination model in response to the input of the partial data.

The additional processing unit 34 executes a process based on the lesion determination information generated by the lesion determination unit 33. For example, based on the lesion determination information, the additional processing unit 34 may execute an automatic diagnosis process for diagnosing a specific lesion state such as the name of the lesion part detected by the lesion determination unit 33 and the degree of the disease. The additional processing unit 34 supplies information (also referred to as "additional processing information") indicating the processing result based on the lesion determination information to the display control unit 35.

It is noted that the additional processing unit 34 may perform processing based on the lesion determination information on the basis of a model configured by referring to parameters previously stored in the memory 12. In this case, for example, the above-mentioned model may be a model trained to output the above-mentioned diagnostic results in response to input of data including the endoscopic image Ia and the lesion determination information. Thus, even in the case of performing processing with high load using the model in the additional processing unit 34, by reducing the amount of calculation in the lesion determination unit 33, it is possible to ensure the real-time processing.

The display control unit 35 generates display information Ib on the basis of the newest endoscopic image Ia supplied from the endoscopic image acquisition unit 30, the lesion determination information supplied from the lesion determination unit 33, and the additional processing information supplied from the additional processing unit 34. Then, the display control unit 35 supplies the generated display information Ib to the display device 2, to thereby display the latest endoscopic image Ia and the lesion detection result or the like on the display device 2. The display example on the display device 2 by the display control unit 35 will be described later. When the display control unit 35 receives the lesion determination information indicating that the lesion part is detected, the display control unit 35 may control the audio output unit 16 to output a warning sound or voice guidance or the like to notify the user that the lesion part is detected.

Each component of the endoscopic image acquisition unit 30, the Fourier transform unit 31, the selection unit 32, the lesion determination unit 33, the additional processing unit 34 and the display control unit 35can be realized, for example, by the processor 11 which executes a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA (Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

### (4-3) Details of Selection Unit

Next, a specific example of the process executed by the selection unit 32. The selection unit 32 selects partial data that is data in a part of the frequency domain on the k-space where the k-space data is present, from the k-space data. In some embodiments, the selection unit 32 uses the k-space data as an image and generates the partial data which is asymmetric with respect to at least either k-x axis or k-y axis, when the center of the image is set as the origin of k-x axis and k-y axis. The k-space is an example of the "frequency space", the k-x axis and the k-y axis are examples of the "first axis" and the "second axis", respectively.

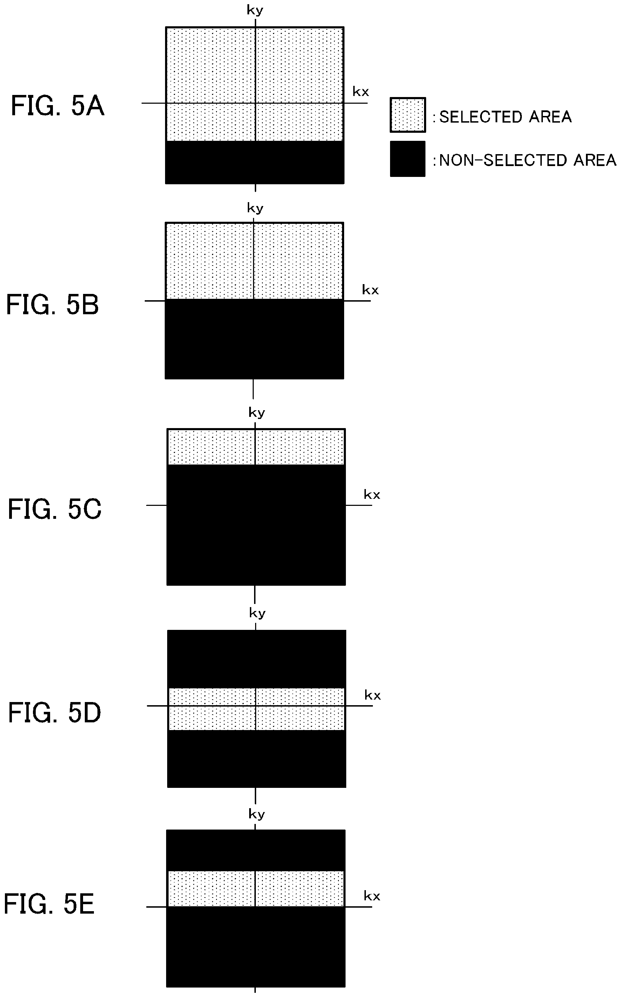

FIG. 5A to 5E show specific examples of explicitly indicating selected areas and non-selected areas in k-space data. In FIGS. 5A to 5E, each hatched area indicates a selected area and each black-painted area indicates a non-selected area. In FIGS. 5A to 5E, the k-x axis and the k-y axis are clearly indicated. Further, FIG. 5A shows an example in which the upper 3/4 range in the k-y axis along the k-space data is used as a selected area, FIG. 5B shows an example in which the upper 1/2 range in the k-y axis along the k-space data is used as a selected area, and FIG. 5C shows an example in which the upper 1/4 range in the k-y axis along the k-space data is used as a selected area. FIG. 5D shows an example in which the center 1/4 range in the k-y axis along the k-space data is set as a selected area, and FIG. 5E shows an example in which the upper 1/4 and lower 1/2 ranges in the k-y axis along the k-space data are set as non-selected areas and the remaining 1/4 range is set as a selected area. Here, each selected area shown in FIGS. 5A to 5C, and 5E is asymmetric with respect to the k-x axis, and the selected area shown in FIG. 5D is symmetric (line symmetry) with respect to the k-x axis. In addition, each selected area shown in FIG. 5A to FIG. 5E is symmetrical (line symmetry) with respect to the k-y axis.

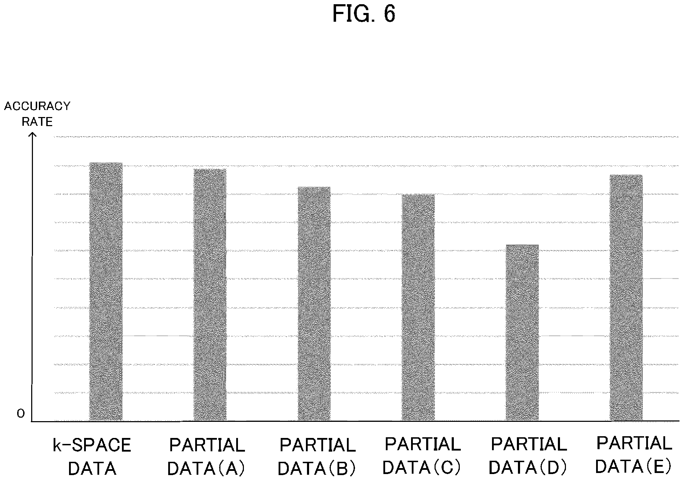

FIG. 6 is a graph showing the accuracy rate in an experiment using partial data shown in FIGS. 5A to 5E in a large bowel endoscopy. In this experiment, each of k-space data and partial data shown in FIG. 5A to FIG. 5E is inputted to a lesion determination model trained to output the degree of inflammation, and the accuracy rate is calculated by comparing the degree of inflammation outputted by the lesion determination model with the correct answer degree of inflammation. In FIG. 6, the "k-space data" indicates the accuracy rate when the data in the whole k-space is used as the input data to the lesion determination model in both the learning stage and the inference stage, and the "partial data (A)" indicates the accuracy rate when the partial data shown in FIG. 5A is used as the input data to the lesion determination model in both the learning stage and the inference stage. Further, the "partial data (B)" indicates the accuracy rate when the partial data shown in FIG. 5B is used as input data to the lesion determination model in both the learning stage and the inference stage, and the "partial data (C)" indicates the accuracy rate when the partial data shown in FIG. 5C is used as input data to the lesion determination model in both the learning stage and the inference stage. The "partial data (D)" indicates the accuracy rate when the partial data shown in FIG. 5D is used as the input data to the lesion determination model in both the learning stage and the inference stage, and the "partial data (E)" indicates the accuracy rate when the partial data shown in FIG. 5E is used as input data to the lesion determination model in both the learning stage and the inference stage.

As shown in FIG. 6, the accuracy rate in the case of using the k-space data is substantially identical to the accuracy rate in the case of using the partial data (A) with reduced 25% data amount. The accuracy rate in the case of using the partial data (E) obtained by reducing the 75% data amount is not significantly different from the accuracy rate in the case of using the k-space data. Thus, the accuracy rate in the case of using the partial data is not significantly different from the accuracy rate in the case of using the k-space data.

The accuracy rate in the cases of using the partial data (A), the partial data (B), the partial data (C), and the partial data (E), which are asymmetric with respect to the k-x axis, are superior to the accuracy rate in the case of using the partial data (D), which is symmetric with respect to the k-x axis. In this way, by setting the selected area so as to be asymmetric with respect to at least one of the k-x axis and/or the k-y axis, it is possible to reduce the amount of data to be inputted to the lesion determination model and reduce the amount of calculation while suppressing the deterioration of the accuracy rate.

Instead of the examples shown in FIGS. 5A to 5E, the selection unit 32 may generate the partial data obtained by setting a part of the range in the k-x axis as the non-selected area. In yet another example, the selection unit 32 may generate partial data in which the non-selected areas for both ranges of the k-x axis and k-y axis.

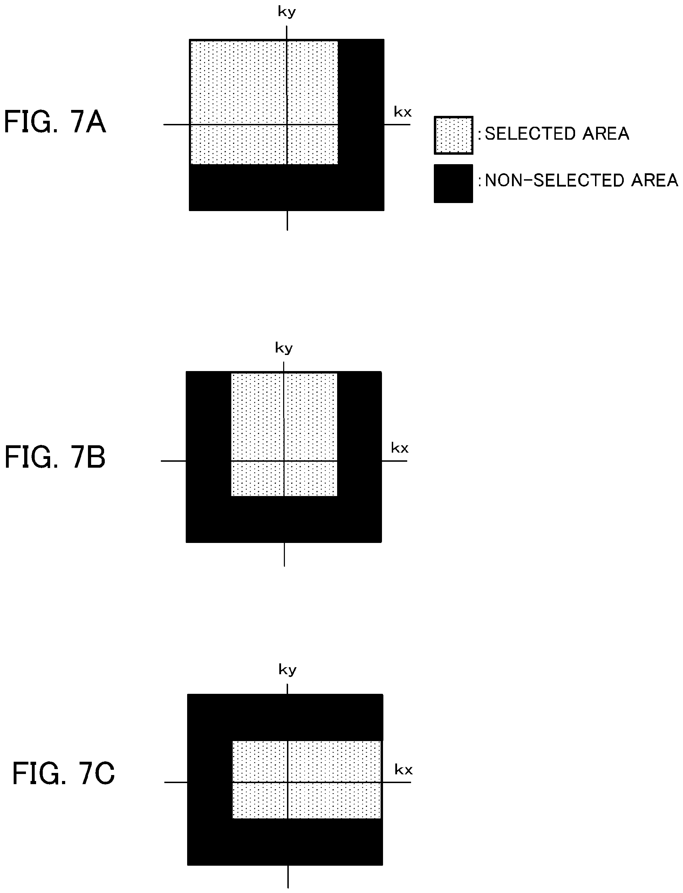

FIG. 7A to FIG. 7C show examples of the k-space data with clear indication of selected areas and non-selected areas when non-selected areas are provided for both partial ranges in the k-x axis and the k-y axis. The partial data shown in FIG. 7A is asymmetrical with respect to both k-x and k-y axes. Further, the partial data shown in FIG. 7B is asymmetric with respect to the k-x axis (and line-symmetric with respect to the k-y axis), and the partial data shown in FIG. 7C is asymmetric with respect to the k-y axis (and line-symmetric with respect to the k-x axis). In these cases, the selected area is set to be asymmetric with respect to at least one of the k-x axis and/or the k-y axis. Therefore, even when these are used as partial data, it is possible to reduce the amount of data to be inputted to the lesion determination model while suppressing the deterioration of the accuracy rate.

### (4-4) Display Example

Next, a description will be given of the display control of the display device 2 to be executed by the display control unit 35.

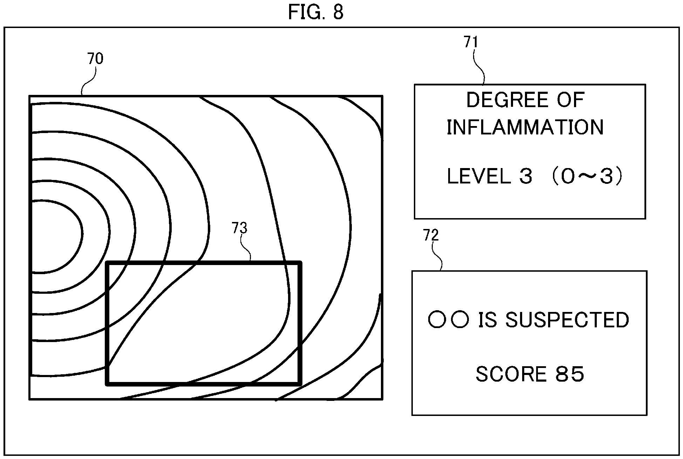

FIG. 8 shows a display example of a display screen image displayed by the display device 2 in the endoscopic examination. The display control unit 35 of the image processing device 1 transmits the display information Ib generated based on the information supplied from the endoscopic image acquisition unit 30, the lesion determination unit 33, and the additional processing unit 34 to the display device 2, thereby causing the display device 2 to display the display screen image shown in FIG.8.

The display control unit 35 of the image processing device 1 displays, on the display screen image, the latest endoscopic image 70, which represents a moving image based on the latest endoscopic image Ia acquired by the endoscopic image acquisition unit 30, the first display field 71 based on the lesion determination information, and the second display field 72 based on the additional processing information.

The display control unit 35 herein displays the contents based on the lesion determination information in the first display field 71. As an example, based on the lesion determination information indicating the determined degree of the inflammation, the display control unit 35 herein displays such information that the inflammation at level 3 on a scale of the level 0 to level 3 has occurred in the first display field 71.

Further, based on the additional processing information, the display control unit 35 displays in the second display field 72 text information that existence of a predetermined disease (here, "∘∘") is suspected and its score (which has a value range of 0 to 100) indicating the degree of reliability of the presence of the above-described disease are displayed. Further, based on the additional processing information, the display control unit 35 displays a frame 73 surrounding the region suspected of the above-described disease on the latest endoscopic image 70.

Thus, the display control unit 35 can notify the examiner of the lesion determination information or the like in real time.

### (4-5) Processing Flow

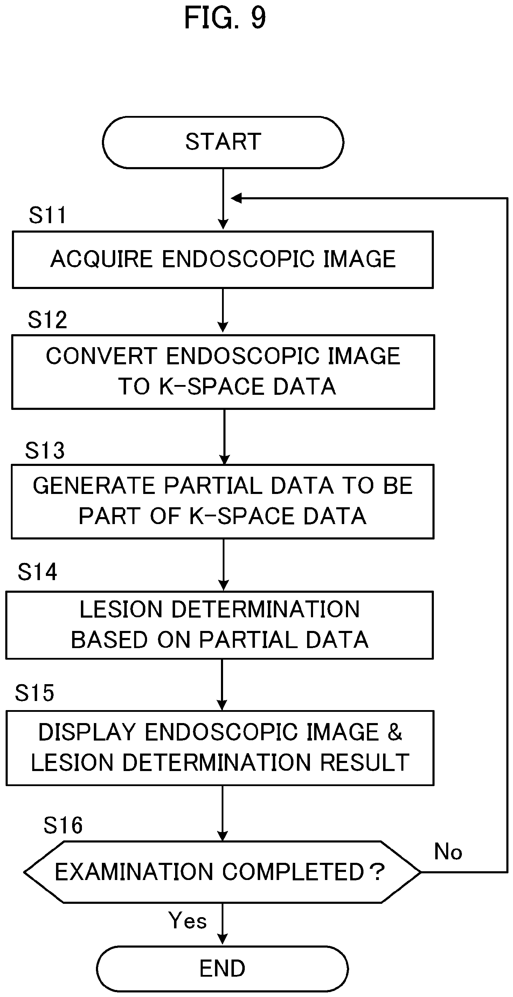

FIG. 9 is an example of a flowchart illustrating an outline of a process that is executed by the image processing device 1 during the endoscopic examination in the first example embodiment.

First, the image processing device 1 acquires an endoscopic image Ia (step S11). In this instance, the endoscopic image acquisition unit 30 of the image processing device 1 receives the endoscopic image Ia from the endoscope 3 through the interface 13.

Next, the image processing device 1 converts the endoscopic image Ia acquired at step S11 into k-space data by Fourier transform (step S12). In this instance, the Fourier transform unit 31 may generate, as the k-space data, absolute value data or phase data into which values of complex numbers of data obtained by applying the Fourier transform to the endoscopic image Ia is converted, and/or, logarithmically converted data.

Then, the image processing device 1 generates partial data which is a part of k-space data (step S13). In this case, for example, the image processing device 1 sets a non-selected area using at least one of k-x axis and/or k-y axis as a reference and generates partial data in which the frequency domain corresponding to the non-selected area is excluded.

Next, the image processing device 1 determines the lesion region in the endoscopic image Ia acquired at step S11 based on the partial data (step S14). The determination made at step S14 may be, for example, a determination regarding the presence or absence of a lesion part in the endoscopic image Ia, or may be a determination of the degree of a particular condition (e.g., inflammation).

Then, the image processing device 1 displays the endoscopic image Ia acquired at step S11 and the lesion determination result acquired at step S14 on the display device 2 (step S15).

Then, the image-processing device 1 determines whether or not the endoscopic examination has been completed (step S16). For example, the image processing device 1 determines that the endoscopic examination has been completed if a predetermined input or the like by the input unit 14 or the operation unit 36 is detected. If it is determined that the endoscopic examination has been completed (Step S16; Yes), the image processing device 1 ends the process of the flowchart. On the other hand, if it is determined that the endoscopic examination has not been completed (step S16; No), the image processing device 1 gets back to the process at step S11. Then, the image processing device 1 performs the processes at step S11 to step S15 on an endoscopic image Ia newly generated by the endoscope 3.

### (5) Modifications

Next, modifications suitable for the above-described example embodiment will be described. The following variations may be applied to the example embodiments described above in combination.

### (First Modification)

The Fourier transform unit 31 of the image processing device 1 may apply the one-dimensional Fourier transform that is a Fourier transform with respect to either the x-axis or y-axis, instead of applying a two-dimensional Fourier transform to the endoscopic image Ia. In this instance, the selection unit 32 provides a non-selected area for a part of the range in the target axis (k-x axis or k-y axis) of the Fourier transform, and generates partial data in which the non-selected area is excluded. In this instance, the data obtained by applying the one-dimensional Fourier transform to the endoscopic image Ia is represented by a space (hybrid-space) having either a set of the k-x axis and the y-axis or a set of the x axis and the k-y axis.

In this mode as well, the image processing device 1 can reduce the amount of data used for the input to the lesion determination model and reduce the amount of calculation related to the lesion determination model.

### (Second Modification)

The image processing device 1 may process the moving image data configured by the captured images Ia generated during endoscopic examination after the examination.

For example, the image processing device 1 sequentially performs the process according to the flowchart in FIG. 9 with respect to each captured image Ia in the time series constituting the moving image data when the moving image data to be processed is specified based on the user input or the like through the input unit 14 at an arbitrary timing after the examination. When it is determined at step S16 that the process of the target moving image data has ended, the image processing device 1 terminates the processing of the flowchart. In contrast, when it is determined that the process of the target moving image data has not ended, the image processing device 1 returns the process to step S11 and performs process according to the flowchart for the next captured image Ia in the time series.

### (Third Modification)

The model information D1 may be stored in a storage device separated from the image processing device 1.

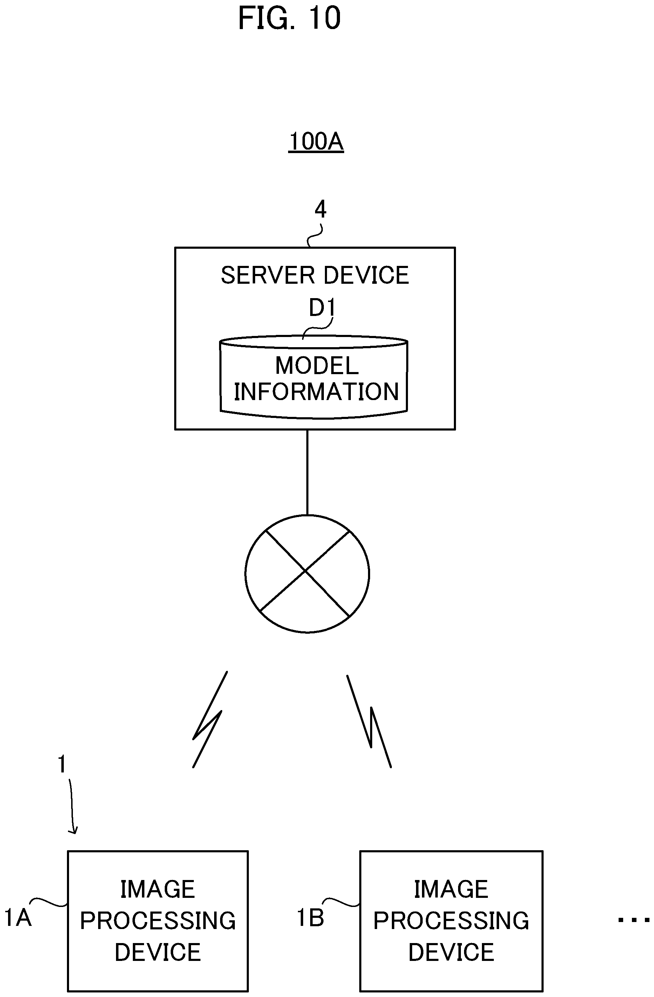

FIG. 10 is a schematic configuration diagram illustrating an endoscopic examination system 100A according to the third modification. For simplicity, the display device 2 and the endoscope 3 and the like are not shown. The endoscopic examination system 100A includes a server device 4 that stores the model information D1. Further, the endoscopic examination system 100A includes a plurality of image processing devices 1 (1A, 1B, ...) capable of data communication with the server device 4 via a network.

In this instance, each image processing device 1 refers to the model information D1 via the network. In this case, the interface 13 of each image processing device 1 includes a communication interface such as a network adapter for performing communication. In this configuration, each image processing device 1 refers to the model information D1 and thereby suitably perform the process relating to the lesion determination as in the above-described example embodiment.

### (Fourth Modification)

The image processing device 1 is not limited to making the determination relating to the lesion part, but may make a determination relating to any attention point (point) which needs to be noticed by the examiner. Examples of such an attention point include a lesion part, an inflammation part, a point with an operating mark or other cuts, a point with a fold or a protrusion, a point on the wall surface of the lumen where the pointed end unit 38 of the endoscope 3 tends to get contact (caught). In this case, the image processing device 1 uses a learned model or the like and makes a determination of whether or not an attention point is present, a determination of the degree regarding the attention point, or the like based on the above-described example embodiment.

The image processing device 1 may determine a coping method (remedy) based on a machine learning model and a determination result of the attention point of the examination target, wherein the model is generated by machine learning of correspondence relation between a determination result (e.g., a determination result indicative of whether an attention point is present or not and a determination result indicative of the degree regarding the attention point) regarding the attention point and the coping method. The above-described model is, for example, a machine learning model trained to output, in response to the input of information relating to a determination result regarding the attention point, an inference result of the coping method corresponding to the inputted determination result. The model information thereof including the learned parameters is previously stored in the memory 12 or the like. The "coping method" is a method of the treatment to be executed by the user (e.g., examiner) according to the determination result regarding the attention point, and examples thereof include instructions of tissue collection for biology.

Then, the image processing device 1 displays information indicating the determined coping method on the display device 2. Instead of the displaying on the display device 2, or in addition to this, the image processing device 1 may output information indicating the coping method by audio output device. The "information indicating the coping method" may be any information (e.g., a name of the coping method (remedy), the identification number, the detailed description, or a combination thereof) for specifying the coping method, for example.

The method of determining the coping method is not limited to the method described above. For example, the image processing device 1 refers to table information and determines the above-described coping method based on the determination result regarding the attention point, wherein the table information indicates a correspondence relation between candidates for the determination result regarding the attention point and a coping method according to each candidate. The table information is stored in advance in the memory 12 or the like.

Thus, it can be used for support a user to perform a decision making.

### <Second Example Embodiment>

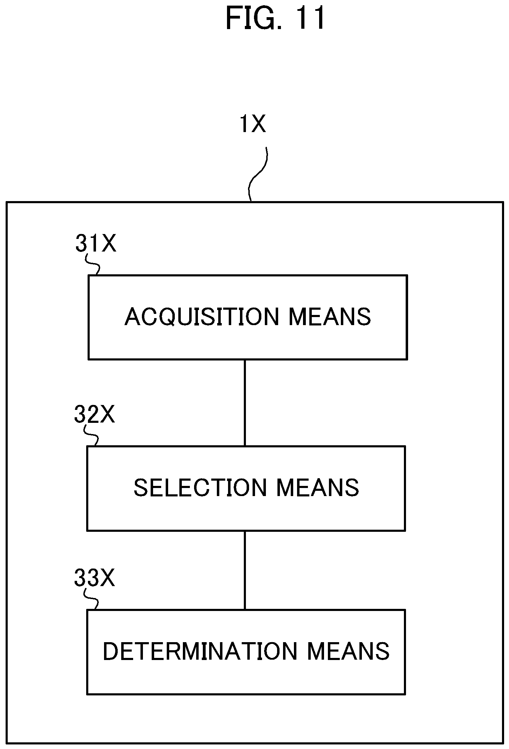

FIG. 11 is a block diagram of an image processing device 1X according to a second example embodiment. The image processing device 1X includes an acquisition means 31X, a selection means 32X, and a determination means 33X. The image processing device 1X may be configured by a plurality of devices.

The acquisition means 31X is configured to acquire data obtained by applying Fourier transform to an endoscopic image of an examination target photographed by a photographing unit provided in an endoscope. Examples of the acquisition means 31X include the Fourier transform unit 31 in the first example embodiment (including modifications, the same shall apply hereinafter). Further, examples of the above-described "data" include k-space data. The Fourier transform is not limited to the two-dimensional Fourier transform and may be the one-dimensional Fourier transform. The acquisition means 31X may acquire the data obtained by applying the Fourier transform to an endoscopic image immediately obtained from the photographing unit, or may acquire above-described data obtained by acquiring at a predetermined timing an endoscopic image stored in the storage device previously generated by the photographing unit in advance and applying the Fourier transform to the acquired data.

The selection means 32X is configured to select partial data that is a part of the data. Examples of the selection means 32X include the selection unit 32 in the first example embodiment.

The determination means 33X is configured to make a determination regarding an attention point to be noticed in the examination target based on the partial data. Examples of the determination means 33X include the lesion determination unit 33 in the first example embodiment.

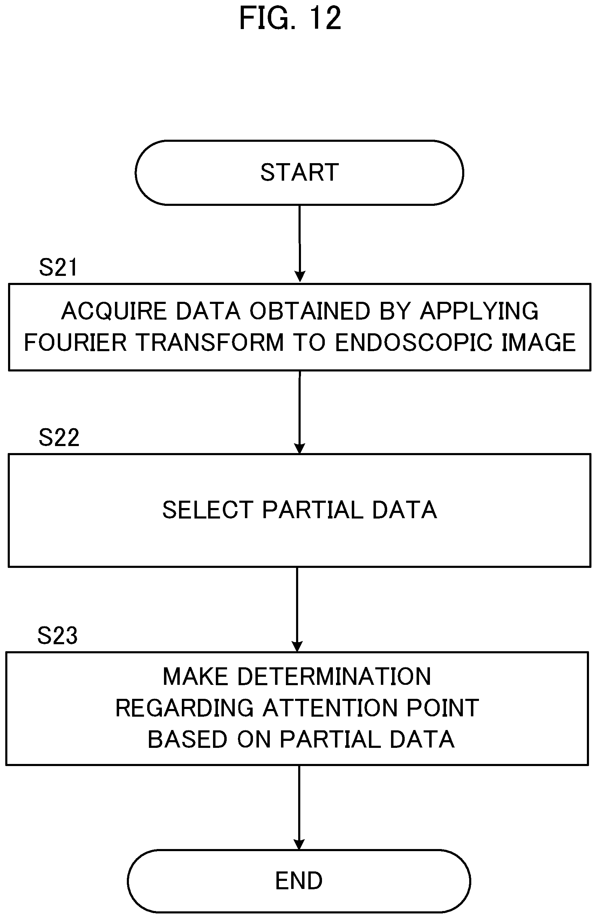

FIG. 12 is an example of a flowchart showing a processing procedure in the second example embodiment. The acquisition means 31X acquires data obtained by applying Fourier transform to an endoscopic image of an examination target photographed by a photographing unit provided in an endoscope (step S21). The selection means 32X selects partial data that is a part of the data (step S22). Then, the determination means 33X makes a determination regarding an attention point to be noticed in the examination target based on the partial data (step S23).

According to the second example embodiment, the image processing device 1X can accurately detect an attention point from an endoscopic image of a photographed examination target.

In the example embodiments described above, the program is stored by any type of a non-transitory computer-readable medium (non-transitory computer readable medium) and can be supplied to a control unit or the like that is a computer. The non-transitory computer-readable medium include any type of a tangible storage medium. Examples of the non-transitory computer readable medium include a magnetic storage medium (e.g., a flexible disk, a magnetic tape, a hard disk drive), a magnetic-optical storage medium (e.g., a magnetic optical disk), CD-ROM (Read Only Memory), CD-R, CD-R/W, a solid-state memory (e.g., a mask ROM, a PROM (Programmable ROM), an EPROM (Erasable PROM), a flash ROM, a RAM (Random Access Memory)). The program may also be provided to the computer by any type of a transitory computer readable medium. Examples of the transitory computer readable medium include an electrical signal, an optical signal, and an electromagnetic wave. The transitory computer readable medium can provide the program to the computer through a wired channel such as wires and optical fibers or a wireless channel.

The whole or a part of the example embodiments described above (including modifications, the same applies hereinafter) can be described as, but not limited to, the following Supplementary Notes.

### [Supplementary Note 1]

An image processing device comprising:
an acquisition means configured to acquire data obtained by applying Fourier transform to an endoscopic image of an examination target photographed by a photographing unit provided in an endoscope;
a selection means configured to select partial data that is a part of the data; and
a determination means configured to make a determination regarding an attention point to be noticed in the examination target based on the partial data.

### [Supplementary Note 2]

The image processing device according to Supplementary Note 1,
wherein the selection means is configured to select the partial data to be asymmetric with respect to at least one of a first axis and/or a second axis in a frequency domain which expresses the data by the first axis and the second axis.

### [Supplementary Note 3]

The image processing device according to Supplementary Note 1,
wherein the determination means is configured to make the determination regarding the attention point, based on the partial data and a model into which the partial data is inputted, and
wherein the model is a machine learning model which learned a relation between
   the partial data to be inputted to the model and
   a determination result regarding the attention point in the endoscopic image used for generation of the partial data.

### [Supplementary Note 4]

The image processing device according to Supplementary Note 1,
wherein the acquisition means is configured to acquire the data obtained by applying two dimensional Fourier transform to the endoscopic image, and
wherein the selection means is configured to generate the partial data in a selected partial range in at least one of the axes to which the Fourier transform is applied.

### [Supplementary Note 5]

The image processing device according to Supplementary Note 1,
wherein the acquisition means is configured to acquire the data obtained by applying one-dimensional Fourier transform to the endoscopic image, and
wherein the selection means is configured to generate the partial data in a selected partial range in the axis to which the Fourier transform is applied.

### [Supplementary Note 6]

The image processing device according to Supplementary Note 1,
wherein the acquisition means is configured to acquire the data that represents an absolute value or a phase into which a complex number for each frequency is converted,
the complex number for each frequency being obtained by applying the Fourier transform to the endoscopic image.

### [Supplementary Note 7]

The image processing device according to Supplementary Note 1,
wherein the acquisition means is configured to acquire the data obtained by applying logarithmic conversion to a value for each frequency,
the value being obtained by applying the Fourier transform to the endoscopic image. [Supplementary Note 8]

The image processing device according to Supplementary Note 1, further comprising
a display control means configured to display information regarding a result of the determination and the endoscopic image on a display device.

### [Supplementary Note 9]

The image processing device according to Supplementary Note 1, further comprising
a coping method determination means configured to determine a coping method based on information regarding a result of the determination and a model into which the information regarding the result of the determination is inputted,
wherein the model is a machine learning model which learned relation between
   information regarding a result of the determination to be inputted to the model and
   the coping method according to the result of the determination.

### [Supplementary Note 10]

An image processing method executed by a computer, the image processing method comprising:
acquiring data obtained by applying Fourier transform to an endoscopic image of an examination target photographed by a photographing unit provided in an endoscope;
selecting partial data that is a part of the data; and
making a determination regarding an attention point to be noticed in the examination target based on the partial data.

### [Supplementary Note 11]

A storage medium storing a program executed by a computer, the program causing the computer to:
acquire data obtained by applying Fourier transform to an endoscopic image of an examination target photographed by a photographing unit provided in an endoscope;
select partial data that is a part of the data; and
make a determination regarding an attention point to be noticed in the examination target based on the partial data.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### DESCRIPTION OF REFERENCE NUMERALS

- 1, 1A, 1X: Image processing device
- 2: Display device
- 3: Endoscope
- 11: Processor
- 12: Memory
- 13: Interface
- 14: Input unit
- 15: Light source unit
- 16: Audio output unit
- 100, 100A: Endoscopic examination system

## Claims

1. An image processing device comprising:
an acquisition means configured to acquire data obtained by applying Fourier transform to an endoscopic image of an examination target photographed by a photographing unit provided in an endoscope;
a selection means configured to select partial data that is a part of the data; and
a determination means configured to make a determination regarding an attention point to be noticed in the examination target based on the partial data.

2. The image processing device according to claim 1,
wherein the selection means is configured to select the partial data to be asymmetric with respect to at least one of a first axis and/or a second axis in a frequency domain which expresses the data by the first axis and the second axis.

3. The image processing device according to claim 1,
wherein the determination means is configured to make the determination regarding the attention point, based on the partial data and a model into which the partial data is inputted, and
wherein the model is a machine learning model which learned a relation between
the partial data to be inputted to the model and
a determination result regarding the attention point in the endoscopic image used for generation of the partial data.

4. The image processing device according to claim 1,
wherein the acquisition means is configured to acquire the data obtained by applying two dimensional Fourier transform to the endoscopic image, and
wherein the selection means is configured to generate the partial data in a selected partial range in at least one of the axes to which the Fourier transform is applied.

5. The image processing device according to claim 1,
wherein the acquisition means is configured to acquire the data obtained by applying one-dimensional Fourier transform to the endoscopic image, and
wherein the selection means is configured to generate the partial data in a selected partial range in the axis to which the Fourier transform is applied.

6. The image processing device according to claim 1,
wherein the acquisition means is configured to acquire the data that represents an absolute value or a phase into which a complex number for each frequency is converted,
the complex number for each frequency being obtained by applying the Fourier transform to the endoscopic image.

7. The image processing device according to claim 1,
wherein the acquisition means is configured to acquire the data obtained by applying logarithmic conversion to a value for each frequency,
the value being obtained by applying the Fourier transform to the endoscopic image.

8. The image processing device according to claim 1, further comprising
a display control means configured to display information regarding a result of the determination and the endoscopic image on a display device.

9. The image processing device according to claim 1, further comprising
a coping method determination means configured to determine a coping method based on information regarding a result of the determination and a model into which the information regarding the result of the determination is inputted,
wherein the model is a machine learning model which learned relation between
information regarding a result of the determination to be inputted to the model and
the coping method according to the result of the determination.

10. An image processing method executed by a computer, the image processing method comprising:
acquiring data obtained by applying Fourier transform to an endoscopic image of an examination target photographed by a photographing unit provided in an endoscope;
selecting partial data that is a part of the data; and
making a determination regarding an attention point to be noticed in the examination target based on the partial data.

11. A storage medium storing a program executed by a computer, the program causing the computer to:
acquire data obtained by applying Fourier transform to an endoscopic image of an examination target photographed by a photographing unit provided in an endoscope;
select partial data that is a part of the data; and
make a determination regarding an attention point to be noticed in the examination target based on the partial data.
